# EUROPEAN PATENT APPLICATION

(11) **EP 3 815 706 A1**
(43) Date of publication of application: **05.05.2021**
(21) Application number: 19825945.9
(22) Date of filing: 07.06.2019
(51) Int. Cl.: A61K 38/14, A61K 47/68, A61P 31/00, A61P 31/04, A61P 31/10, A61P 31/12, A61P 33/00, A61P 43/00

(54) **AGENT FOR ENHANCING PHAGOCYTOSIS ABILITY OF NEUTROPHILS**

(30) Priority: 28.06.2018 JP 2018123626
(71) Applicant: National University Corporation Okayama University, Kita-ku Okayama-shi Okayama 700-8530 (JP)
(72) Inventor: NISHIBORI, Masahiro, Okayama-shi, Okayama 700-8530 (JP); WAKE, Hidenori, Okayama-shi, Okayama 700-8530 (JP); TAKAHASHI, Youhei, Okayama-shi, Okayama 700-8530 (JP); MORI, Shuji, Okayama-shi, Okayama 700-8530 (JP); SAKAGUCHI, Masakiyo, Okayama-shi, Okayama 700-8530 (JP)
(74) Representative: Hertin und Partner Rechts- und Patentanwälte PartG mbB
(86) International application number: PCT/JP2019/022812
(87) International publication number: WO 2020/003978

(57) **Abstract**

It is an object of the present invention to provide an agent for enhancing phagocytosis ability, specifically an agent for enhancing phagocytosis ability that functions as a therapeutic agent or therapeutic adjunct for various bacterial infectious diseases, viral infectious diseases, fungal infectious diseases, parasitic infectious diseases, and mixed infectious diseases thereof by enhancing phagocytosisability of neutrophils. The above-mentioned object is achieved by an agent for enhancing phagocytosis ability including HRG as an active ingredient. The inventors of the present invention have focused attention on the function of HRG on regulation of neutrophil activities, and as the result of extensive investigations, have newly found that HRG effectively enhances phagocytosis ability against a pathogen or foreign matter derived from bacteria, viruses, fungi, parasites, and the like among the neutrophil activities.

## Description

### Technical Field

The present invention relates to an agent for enhancing phagocytosis ability of neutrophils, and more particularly, to an agent for enhancing phagocytosis ability of neutrophils including a histidine-rich glycoprotein as an active ingredient.

The present application claims priority from Japanese Patent Application No. 2018-123626, which is incorporated herein by reference.

### Background Art

Neutrophils have migration, phagocytosis, and microbicidal functions. Phagocytosis ability (phagocytosis) is the first barrier of living bodies against microbe invasion, and is regarded as important as a fundamental function of immune systems. The main role of neutrophils is a biological defense function: phagocytosing foreign matter, typically pathogenic microbes invading living bodies; and killing the microbes with reactive oxygen species generated therefrom. Neutrophils have non-specific immune ability to eliminate substances that invade living bodies. When immunoglobulins, complements, or the like bind to foreign matter to cause opsonization (Op), neutrophils recognize and phagocytose the foreign matter. However, no therapeutic agent for enhancing bacterium phagocytosis activity has been known. There has been demanded a method for ameliorating a bacterium clearance disorder due to lowered bacterium phagocytosis ability of neutrophils.

Histidine-rich glycoprotein (HRG) is a plasma protein having a molecular weight of about 80 kDa, which was identified by Heimburger et al. (1972). HRG is a histidine-rich protein formed of a total of 507 amino acids including 66 histidines, is mainly synthesized in the liver, and is present in human plasma at a concentration of from about 100 µg/mL to about 150 µg/mL, which is regarded as extremely high. It is known that HRG is involved in regulation of coagulation and fibrinolytic systems and regulation of angiogenesis (Non Patent Literature 1). Further, there are disclosures of: methods for the inhibition of angiogenesis by administering an HRG polypeptide; and pharmaceutical compositions and articles of manufacture including HRG polypeptides, antibodies and receptors that bind to an HRG polypeptide, HRG-depleted plasma and polynucleotides, vectors and host cells that encode HRG polypeptides (Patent Literature 1). In addition, there is a disclosure relating to the field of angiogenesis, and more particularly to the use of a substantially pure consecutive polypeptide having anti-angiogenic activity, including subfragments derived from a central region of HRG (Patent Literature 2). Further, there is also a disclosure of a depressant agent for neutrophil-vascular endothelial cell adhesion, containing HRG as an active ingredient (Patent Literature 3). In Patent Literature 3, there is a disclosure of a regulatory action on neutrophil activation including HRG as an active ingredient. However, bacterium phagocytosis activity of neutrophils and the like has not been reported.

There is a disclosure that a protein of interest was generated using a gene expression cassette of a special structure (PatentLiterature4). In Patent Literature 4, there is a description that cells capable of stably and highly producing the protein of interest are obtained through use of the gene expression cassette, and as examples of the generated protein, HRG, an HRG-Fc fusion protein, and the like are described in Examples. Fusion products of an Fc domain of IgG with specific substances, such as proteins classified into receptors, cytokines, peptides, and enzymes, are known, which have artificially been designed expecting a prolonged half-life in blood. However, the action of an HRG-Fc fusion protein has not been reported yet.

### Citation List

### Non Patent Literature

[NPL 1] Blood, Vol. 117, No. 7, 2093-2101 (2011)

### Patent Literature

[PTL 1] JP 2004-527242 A
[PTL 2] JP 2007-528710 A
[PTL 3] JP 5807937 B2 (WO 2013/183494 A1)
[PTL 4] WO 2017/061354 A1

### Summary of Invention

### Technical Problem

An object of the present invention is to provide an agent for enhancing phagocytosis ability of neutrophils. Another object of the present invention is to provide an agent for enhancing phagocytosis ability of neutrophils as a therapeutic agent or therapeutic adjunct for various bacterial infectious diseases, viral infectious diseases, fungal infectious diseases, parasitic infectious diseases, and mixed infectious diseases thereof.

### Solution to Problem

The present invention is directed to an agent for enhancing phagocytosis ability of neutrophils, including HRG as an active ingredient. To solve the above-mentioned objects, the inventors of the present invention have focused attention on HRG having a regulatory function of neutrophil activities, and as the result of extensive investigations, have newly found that HRG exhibits an enhancing effect of phagocytosis ability against pathogens or foreign matter caused by bacteria, viruses, fungi, parasites, and the like among the neutrophil activities, thus completing the present invention.

That is, the present invention includes the following.
1. An agent for enhancing phagocytosis ability of neutrophils, including a histidine-rich glycoprotein as an active ingredient.
2. The agent for enhancing phagocytosis ability of neutrophils according to the above-mentioned item 1, wherein the histidine-rich glycoprotein, which is the active ingredient, is present in the form of an Fc-fusion protein including the histidine-rich glycoprotein fused with an Fc region of an antibody.
3. The agent for enhancing phagocytosis ability of neutrophils according to the above-mentioned item 2, wherein the Fc region of an antibody is an Fc region derived from IgG₂.
4. The agent for enhancing phagocytosis ability of neutrophils according to any one of the above-mentioned items 1 to 3, wherein the agent serves as a therapeutic agent for an infectious disease or a therapeutic adjunct for an infectious disease.
5. The agent for enhancing phagocytosis ability of neutrophils according to the above-mentioned item 4, wherein the infectious disease is an infectious disease caused by a pathogen derived from one or a plurality of species selected from bacteria, viruses, fungi, and parasites.
6. The agent for enhancing phagocytosis ability of neutrophils according to the above-mentioned item 4 or 5, wherein the infectious disease is any one of infectious diseases selected from a respiratory infectious disease, a urinary tract infectious disease, a biliary tract infectious disease, a gastrointestinal infectious disease, and a central nervous system infectious disease.
   A. A method of enhancing phagocytosis ability of neutrophils using HRG as an active ingredient of an agent for enhancing phagocytosis ability of neutrophils.
   B. A therapeutic method for an infectious disease or an adjunctive therapeutic method for an infectious disease using HRG as an active ingredient of an agent for enhancing phagocytosis ability of neutrophils.
   C. The method of enhancing phagocytosis ability of neutrophils according to the above-mentioned item A, or the therapeutic method for an infectious disease or the adjunctive therapeutic method for an infectious disease according to the above-mentioned item B, wherein the HRG, which is the active ingredient, is in the form of an Fc-fusion protein including the HRG fused with an Fc region of an antibody.
   D. The method of enhancing phagocytosis ability of neutrophils, or the therapeutic method for an infectious disease or the adjunctive therapeutic method for an infectious disease according to the above-mentioned item C, wherein the Fc region of an antibody is an Fc region derived from IgG₂.
   E. The therapeutic method for an infectious disease or the adjunctive therapeutic method for an infectious disease according to the above-mentioned item D, wherein the infectious disease is an infectious disease caused by a pathogen derived from one or a plurality of species selected from bacteria, viruses, fungi, and parasites.
   F. The therapeutic method for an infectious disease or the adjunctive therapeutic method for an infectious disease according to the above-mentioned item D or E, wherein the infectious disease is any one of infectious diseases selected from a respiratory infectious disease, a urinary tract infectious disease, a biliary tract infectious disease, a gastrointestinal infectious disease, and a central nervous system infectious disease.

### Advantageous Effects of Invention

The agent for enhancing phagocytosis ability of neutrophils including HRG as an active ingredient according to the present invention (hereinafter sometimes abbreviated as "agent for enhancing phagocytosis ability of neutrophils" of the present invention) has an action of effectively enhancing phagocytosis ability of neutrophils against foreign matter, such as a bacterium, upon having been caused to act on neutrophils in a normal condition.

### Brief Description of Drawings

FIG. 1 is a view for illustrating a structure of an HRG-carrying construct required for producing recombinant human HRG. (Example 2)
FIG. 2 is a view for illustrating a DNA base sequence encoding an HRG-coding region required for producing recombinant human HRG or an HRG-Fc fusion protein (EcoR1-HRG-Xho1: SEQ ID NO: 3). (Example 2)
FIG. 3 is a view for illustrating a base sequence encoding an FC region of human IgG₂ required for producing an HRG-Fc fusion protein (XhoI-GPG-hIgG₂ Fc-Xba1: SEQ ID NO: 4). (Example 3)
FIGS. 4 are photographs and graphs showing results of analysis of phagocytosis ability of neutrophils at different concentrations of HRG against *Escherichia coli* and *Staphylococcus aureus.* (Experimental Example 1)
FIG. 5 includes photographs showing results of image analysis of phagocytosis ability of neutrophils at different concentrations of hHRG, rHRG, or HRG-Fc in phagocytosis of neutrophils against *Escherichia coli.* (Experimental Example 2)
FIG. 6 includes photographs showing results of image analysis of phagocytosis ability of neutrophils by hHRG, rHRG, or HRG-Fc or a control, such as HBSS or HSA, in phagocytosis of neutrophils against *Escherichia coli.* (Experimental Example 2)
FIG. 7 is a graph showing results of analysis of phagocytosis ability of neutrophils by hHRG, rHRG, or HRG-Fc in phagocytosis of neutrophils against *Escherichia coli.* (Experimental Example 2)
FIG. 8 includes photographs showing results of image analysis of phagocytosis ability of neutrophils by hHRG, rHRG, or HRG-Fc at different concentrations in phagocytosis of neutrophils against *Staphylococcus aureus.* (Experimental Example 2)
FIG. 9 includes photographs showing results of image analysis of phagocytosis ability of neutrophils by hHRG, rHRG, or HRG-Fc or a control, such as HBSS or HSA, in phagocytosis of neutrophils against *Staphylococcus aureus.* (Experimental Example 2)
FIG. 10 is a graph showing results of analysis of phagocytosis ability of neutrophils by hHRG, rHRG, or HRG-Fc in phagocytosis ability of neutrophils against *Staphylococcus aureus.* (Experimental Example 2)
FIG. 11 includes photographs showing results of analysis of phagocytosis ability of neutrophils by IL-8, fMLP, LPS, C5a, or the like in phagocytosis ability of neutrophils against *Escherichia coli.* (Comparative Example 1)
FIG. 12 includes photographs showing results of analysis of phagocytosis ability of neutrophils by IL-8, fMLP, LPS, C5a, or the like in phagocytosis ability of neutrophils against *Staphylococcus aureus.* (Comparative Example 1)
FIG. 13 includes graphs and photographs showing results of tests observing the enhancing action of HRG stimuli on phagocytosis ability of neutrophils. FIG. 13 includes graphs and photographs showing results observing the action of HRG using anti-HRG antibodies in phagocytosis ability of neutrophils against *Escherichia coli* or *Staphylococcus aureus.* (Experimental Example 3)
FIG. 14 is a graph showing a result observing an effect of HRG on opsonization (Op), where neutrophils phagocytose opsonized (Op) Zymosan by using serum containing HRG or containing no HRG. (Experimental Example 4)
FIG. 15 illustrates graphs showing results observing the difference of phagocytosis ability of neutrophils against *Escherichia coli* or *Staphylococcus aureus* depending on the presence and absence of HRG, the neutrophils having been pretreated with HRG or 5-fold-diluted human sera (opsonized (Op) when human sera are used). FIG. 15A shows phagocytosis actions of neutrophils against *Escherichia coli,* and FIG. 15B shows phagocytosis actions of neutrophils against *Staphylococcus aureus.* (Experimental Example 5)
FIG. 16 illustrates photographs showing phagocytosis actions of neutrophils against *Escherichia coli* or *Staphylococcus aureus* pretreated with 5-fold-diluted human serum. (Experimental Example 5)

### Description of Embodiments

The present invention relates to an agent for enhancing phagocytosis ability of neutrophils, including HRG as an active ingredient.

The main role of neutrophils is a biological defense function: phagocytosing foreign matter, typically pathogenic microbes invading living bodies; and killing the microbes with reactive oxygen species generated therefrom. Neutrophils have non-specific immune ability to eliminate substances that invade living bodies. When immunoglobulins, complements, or the like bind to foreign matter to cause opsonization (Op), neutrophils recognize and phagocytose the foreign matter. However, no therapeutic agent for enhancing a bacterium phagocytosis activity of neutrophils has been known. There has been demanded a method for ameliorating a bacterium clearance disorder due to lowered bacterium phagocytosis ability of neutrophils.

The HRG, included as the active ingredient in the "agent for enhancing phagocytosis ability of neutrophils" of the present invention, can be prepared by a method for isolation or purification from a biological component, a method for preparation using a recombination technology, or synthesis; and the purity thereof is not particularly limited to the maximum purity. The HRG can be subjected to, for example, purification and/or isolation from a biological component such as: blood, for example, plasma or serum; spinal fluid; or lymphatic fluid. The biological component is suitably a blood component, such as plasma or serum. A method known *per* se or any method to be developed in the future may be applied as the method for the isolation/purification from a biological component. For example, the HRG may be prepared by passing plasma through an affinity column prepared using a nickel-nitrilotriacetic acid (Ni-NTA) agarose resin.

A method known *per* se or any method to be developed in the future may be applied as the method for the preparation of HRG using a recombination technology. For example, the preparation can be performed by cloning the full-length cDNA encoding HRG or a cDNA encoding a part having the activity of HRG into an expression vector. For example, a protein biosynthesized from the whole or a part of the nucleotide defined by GenBank Accession No. NM000412 can be used. For example, the preparation can be performed by cloning the full-length cDNA encoding the amino acid sequence of mature HRG (SEQ ID NO: 1) or a cDNA encoding a part thereof into an expression vector. The HRG included as the active ingredient in the "agent for enhancing phagocytosis ability of neutrophils" of the present invention can optionally be an HRG protein itself, or a part of a protein or peptide having the activity of HRG. The HRG can be with or without a carbohydrate chain. The mature HRG, following truncation of its signal peptide by a proteinase, includes four principal regions: (1) cystatin-like region 1, (2) cystatin-like region 2, (3) a His/Pro region, and (4) a C-terminal region. The His/Pro region is extremely rich in proline residues and histidine residues, and can be defined by the amino acid sequence shown as amino acids 330 to 389 of the amino acid sequence set forth in SEQ ID NO: 1.

Amino Acid Sequence of Mature HRG (SEQ ID NO: 1)

VSPTDCSAVEPEAEKALDLINKRRRDGYLFQLLRIADAHLDRVENTTVYYLVLDVQESDCSV

LSRKYWNDCEPPDSRRPSEIVIGQCKVIATRHSHESQDLRVIDFNCTTSSVSSALANTKDSP

VLIDFFEDTERYRKQANKALEKYKEENDDFASFRVDRIERVARVRGGEGTGYFVDFSVRNCP

RHHFPRHPNVFGFCRADLFYDVEALDLESPKNLVINCEVFDPQEHENINGVPPHLGHPFHWG

GHERSSTTKPPFKPHGSRDHHHPHKPHEHGPPPPPDERDHSHGPPLPQGPPPLLPMSCSSCQ

HATFGTNGAQRHSHNNNSSDLHPHKHHSHEQHPHGHHPHAHHPHEHDTHRQHPHGHHPHGHH

PHGHHPHGHHPHGHHPHCHDFQDYGPCDPPPHNQGHCCHGHGPPPGHLRRRGPGKGPRPFHC

RQIGSVYRLPPLRKGEVLPLPEANFPSFPLPHHKHPLKPDNQPFPQSVSESCPGKFKSGFPQ

VSMFFTHTFPK

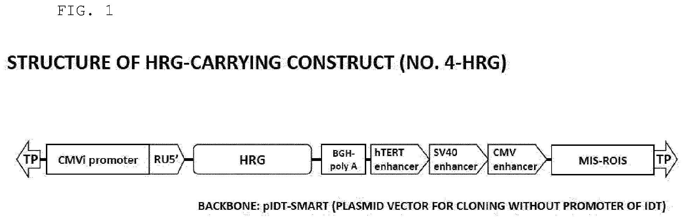

Specifically, a method described in Patent Literature 4 can be applied as a method of generating HRG through use of a recombination technology. For example, a full-length cDNA encoding HRG, or cDNA encoding a part having the activity of HRG, such as a full-length cDNA encoding the amino acid sequence (SEQ ID NO: 1) of the mature HRG, or a cDNA encoding a part thereof, is cloned into an expression vector to prepare a desired HRG expression vector. The HRG protein can be prepared by, for example, using an HRG-carrying construct illustrated in FIG. 1.

To prepare an HRG-Fc fusion protein, it is suitable to bind a cDNA encoding Fc to a portion encoding the C'-terminal region of HRG. An HRG protein included in the HRG-Fc fusion protein of the present invention can be the whole mature HRG, or those with or without a carbohydrate chain, as well as a part of a protein or peptide having the activity of HRG out of the mature HRG.

The Fc included in the HRG-Fc fusion protein can be any polypeptide defining an Fc region of an antibody. The antibody includes polypeptides called a heavy chain (H chain) and a light chain (L chain). The H chain includes a variable region (VH) and a constant region (CH) from its N-terminus, and the L chain includes a variable region (VL) and a constant region (CL) from its N-terminus. The CH includes domains CH1, hinge, CH2, and CH3 from its N-terminus. The region including both CH2 and CH3 is called an Fc region. Examples of classes of the antibody include IgG, IgA, IgE, and IgM. Examples of subclasses of the IgG include IgG₁, IgG₂, IgG₃, and IgG₄. The Fc region used for the HRG-Fc fusion protein of the present invention can be derived from any of the subclasses IgG₁, IgG₂, IgG₃, and IgG₄, particularly suitably IgG₂. The use of the Fc region of IgG₂ can lead to be low complement activity, ameliorating a side effect inflammatory reaction. The amino acid sequence of the Fc region of human IgG₂ is set forth in SEQ ID NO: 2.

Amino Acid Sequence of Fc Region of Human IgG₂ (SEQ ID NO: 2)

ERKCCVECPPCPAPPVAGPSVFLFPPKPKDTLMISRTPEVTCVVVDVSHEDPEVQFNWYVDG

VEVHNAKTKPREEQFNSTFRVVSVLTVVHQDWLNGKEYKCKVSNKGLPAPIEKTISKTKGQP

REPQVYTLPPSREEMTKNQVSLTCLVKGFYPSDISVEWESNGQPENNYKTTPPMLDSDGSFF

LYSKLTVDKSRWQQGNVFSCSVMHEALHNHYTQKSLSLSPG

Proteins, peptides, and the like, which are generally believed to be less stable and less efficient in living bodies, may be fused with an Fc region, achieving improved stability and efficacy in a formulation. The use of the Fc region of human IgG₂ allows a pharmaceutical formulation having a lower complement activity and an ameliorated side effect inflammatory reaction.

The "agent for enhancing phagocytosis ability of neutrophils" of the present invention can enhance the bacterium phagocytosis activity of neutrophils and the like. The above-mentioned "agent for enhancing phagocytosis ability of neutrophils" of the present invention may include HRG as an active ingredient, and may include, for example, HRG *per se* and/or an HRG-Fc fusion protein as well as a pharmacologically acceptable carrier. Examples of the pharmacologically acceptable carrier may include excipients, disintegrants or disintegration aids, binders, lubricating agents, coating agents, dyes, diluents, bases, solubilizing agents or dissolution aids, tonicity agents, pH adjusters, stabilizers, propellants, and pressure-sensitive adhesives.

The "agent for enhancing phagocytosis ability of neutrophils" of the present invention can enhance phagocytosis ability of neutrophils against a pathogen or foreign matter derived from bacteria, viruses, fungi, or parasites. Thus, the "agent for enhancing phagocytosis ability of neutrophils" can be used for a therapeutic agent or therapeutic adjunct for a bacterial infectious disease, a viral infectious disease, a fungal infectious disease, a parasitic infectious disease, and mixed infectious diseases thereof. Examples of the infectious diseases applicable to the present invention may include the above-mentioned various infectious diseases, and may also include a respiratory infectious disease, a urinary tract infectious disease, a biliary tract infectious disease, a gastrointestinal infectious disease, and a central nervous system infectious disease. The present invention encompasses a therapeutic agent and therapeutic adjunct for such diseases.

The present invention also encompasses a method of enhancing phagocytosis ability of neutrophils using HRG as an active ingredient of an agent for enhancing phagocytosis ability of neutrophils. The present invention also encompasses a therapeutic method of an infectious disease or an adjunctive therapeutic method for an infectious disease using HRG as an active ingredient of an agent for enhancing phagocytosis ability of neutrophils.

The "agent for enhancing phagocytosis ability of neutrophils" of the present invention, when used as a therapeutic agent or therapeutic adjunct for the above-mentioned diseases, may topically or systemically be administered. Dosage forms for therapeutic and/or preventive agents may include enteral administration formulations and parenteral administration formulations. Examples of the parenteral administration formulations may include external preparations for transdermal administration, such as a lotion, an ointment, a tape, and a plaster, as well as those in the form suitable for topical administration by injection and the like, intravenous injection administration, and transluminal administration. The parenteral administration formulations may include a sterilized aqueous or nonaqueous solution, suspension, or emulsion. Examples of nonaqueous diluents are propylene glycol, polyethylene glycol, plant oils, such as olive oil, and organic ester compositions, such as ethyl oleate, which are suitable for injection. Aqueous carriers may include water, alcoholic/aqueous solutions, emulsions, suspensions, saline, and buffered media. Parenteral carriers may include sodium chloride solution, Ringer's dextrose, dextrose and sodium chloride, lactated Ringer's, and fixed oils. Parenteral administration formulations in the form suitable for topical administration by injection or the like, intravenous injection administration, or intraluminal administration may include fillers for liquid, nutrients (including monosaccharides, oligosaccharides, polysaccharides, starches, or partial starch hydrolyzates) or electrolytes (e.g., those based on Ringer dextrose) as carriers. The "agent for enhancing phagocytosis ability of neutrophils" of the present invention may include a preservative and other additives, for example, an antimicrobial compound, an antioxidant, a chelating agent, and an inert gas.

When used as the therapeutic agent or therapeutic adjunct for the diseases described above, the "agent for enhancing phagocytosis ability of neutrophils" of the present invention can be administered in combination with any other pharmaceutical formulation. The other pharmaceutical formulation that can be used in combination should not be particularly limited, and may generally be any already commercially available therapeutic agent or preventive agent, or any therapeutic agent or preventive agent to be developed in the future. The other pharmaceutical formulation is suitably a pharmaceutical formulation having an action mechanism different from that of the "agent for enhancing phagocytosis ability of neutrophils" of the present invention. The "agent for enhancing phagocytosis ability of neutrophils" of the present invention can be used before, during or after use of a therapeutic agent and/or a preventive agent for an infectious disease known *per se,* including, for example, antibiotic agents, antimicrobial agents, and viral agents. The "agent for enhancing phagocytosis ability of neutrophils" of the present invention can be used in combination with any other pharmaceutical formulation to lower the dose of the other pharmaceutical formulation. When the other pharmaceutical formulation has a side effect, the combined use with the "agent for enhancing phagocytosis ability of neutrophils" of the present invention can reduce the dose/administration frequency of the other pharmaceutical formulation, achieving a practical advantage of reducing the side effect of the other pharmaceutical formulation. Each ingredient other than HRG as an active ingredient may be blended in any appropriate amounts that do not impair an intended purpose of the present invention.

### Examples

The present invention is specifically described by way of the following Examples, experimental examples, and comparative examples, but it goes without saying that the present invention is not limited by these Examples and the like.

### (Example 1) Purification of Human Plasma-derived HRG

Human plasma was used as a starting raw material. Human plasma-derived HRG (hereinafter referred to as "hHRG") was purified using Ni-NTA affinity chromatography and high performance liquid chromatography (anion exchange column (monodisperse hydrophilic polymer beads: Mono Q)). A purified hHRG sample was obtained in the molecular weight fraction of about 80 kDa. hHRG of this Example was generated by a method described in Example 1 of Patent Literature 3 (WO 2013/183494 A1). hHRG samples having various concentrations were generated through dilution with Hanks' solution (Hanks' Balanced Salt Solution: HBSS).

### (Example 2) Production of Recombinant Human HRG

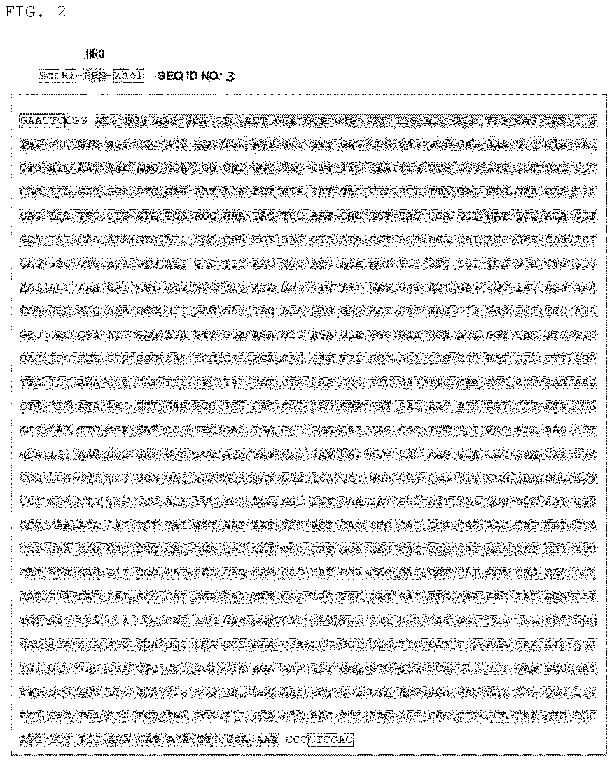

Recombinant human HRG (hereinafter referred to as "rHRG") of this Example was generated by a method described in Example 3 of Patent Literature 4 (WO 2017/061354 A1). Into Chinese Hamster Ovary cells (CHO cells) cultured in 10% FCS-containing GIBCO⁽™⁾ Dulbecco's Modified Eagle Medium/ Nutrient Mixture F-12 (DMEM/F12), an HRG expression vector, a transposase expression vector, and a drug-resistant gene expression vector (see FIG. 1) were co-transfected. After the transfection, 10 µg/mL of Puromycin (an antibiotic) was added to the culture after 48 hours culturing to perform drug selection culture for 3 weeks while exchanging the medium once every three days. A DNA base sequence encoding the HRG-coding region (EcoR1-HRG-Xho1) required for generation of recombinant human HRG is set forth in SEQ ID NO: 3 (FIG. 2).

After the selection culture, rHRG-containing culture supernatant was recovered. To the culture supernatant, a QIAGEN⁽™⁾ Ni-NTA agarose gel prewashed with 30 mL of PBS(-) (a gel in which Ni-NTA was bound to Sepharose CL-6B support) was added, and the resulting culture was subjected to rotation incubation at 4°C for 2 hours, allowing the rHRG to bind to the QIAGEN⁽™⁾ Ni-NTA agarose gel. After the QIAGEN⁽™⁾ Ni-NTA agarose gel was transferred to a column for purification, the column was sequentially washed with washing liquid 1 (30 mM imidazole-containing PBS(-) (pH 7.4)), washing liquid 2 (1 M NaCl + 10 mM PBS (pH 7.4)), and washing liquid 3 (PBS(-) (pH 7.4)). The rHRG was eluted with 500 mM imidazole-containing PBS(-) (pH 7.4) at 4°C. rHRG samples having different concentrations were prepared by dilution with HBSS.

### (Example 3) Generation of HRG-Fc Fusion Protein

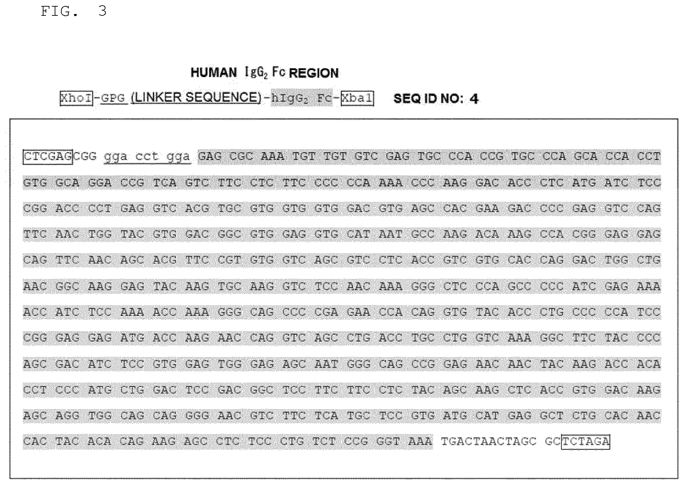

In this Example, a fusion protein of HRG + Fc of human IgG₂ (hereinafter referred to as "HRG-Fc") was generated. To a nucleic acid including the base sequence defined by the DNA encoding an HRG-coding region (GenBank Accession No. BC069574 (NCBI)), a nucleic acid including the base sequence encoding an Fc region of human IgG₂ was bound to generate the HRG-Fc by a method described in Example 12 of Patent Literature 4 (WO 2017/061354A1). HRG-Fc samples having different concentrations were prepared by dilution using HBSS. In the same manner as in Example 2, a DNA base sequence encoding an HRG-coding region (EcoR1-HRG-Xho1) is set forth in SEQ ID NO: 3 (FIG. 2), and the base sequence encoding the Fc region of human IgG₂ (XhoI-GPG-hIgG₂ Fc-Xba1) is set forth in SEQ ID NO: 4 (FIG. 3). Pharmaceutical candidate proteins are all conceived to improve the stability of protein formulations through fusion to partial antibodies, human IgG Fc regions, allowing pharmaceuticals having lowered complement activity and ameliorated side effect inflammatory reaction by using additionally the human IgG₂ Fc regions.

### (Experimental Example 1) Observation Test 1 on Phagocytosis Ability of Neutrophils with HRG Stimulus

Phagocytosis ability of neutrophils against *Escherichia coli* (a gram-negative bacterium) or *Staphylococcus aureus* (a gram-positive bacterium) was measured with a pHrodo™ indicator. Phagosomes within neutrophils can be fluorescence-stained with a pHrodo™ staining reagent to measure their phagocytosis ability. Human neutrophils were separated from the whole blood using a hemocyte separation solution Polymorphprep™, and the cell solution was washed with PBS to perform suspension. The numbers of cells were adjusted, and the cells were stained with Hoechst33342 (nucleus staining: blue) and calcein-AM (cytoplasm: green) by incubation under CO₂ at 37°C for 15 min. Neutrophil solutions were resuspended in HBSS, and the numbers of cells were adjusted (2×10⁶ cells/mL).

To the neutrophil liquids at the cell concentration, hHRG solutions were added, and the mixtures were dispensed to a 96-well plate, allowing incubation under CO₂ at 37°C for 30 min. Then, *Escherichia coli* (pHrodo™ *Escherichia coli)* or *Staphylococcus aureus* (pHrodo™ *Staphylococcus aureus)* was added to the 96-well plate, and incubated under CO₂ at 37°C. In this Example, the final concentration of the cell liquids was 2×10⁴ cells/well (2×10⁵ cells/mL), the final concentrations of HRG were from 0.01 µM to 3.0 µM, and the final concentration of pHrodo™ *Escherichia coli* or pHrodo™ *Staphylococcus aureus* was 25 µg/well (250 µg/mL). The incubation of *Escherichia coli* was for 60 min, and that of *Staphylococcus aureus* was for 120 min.

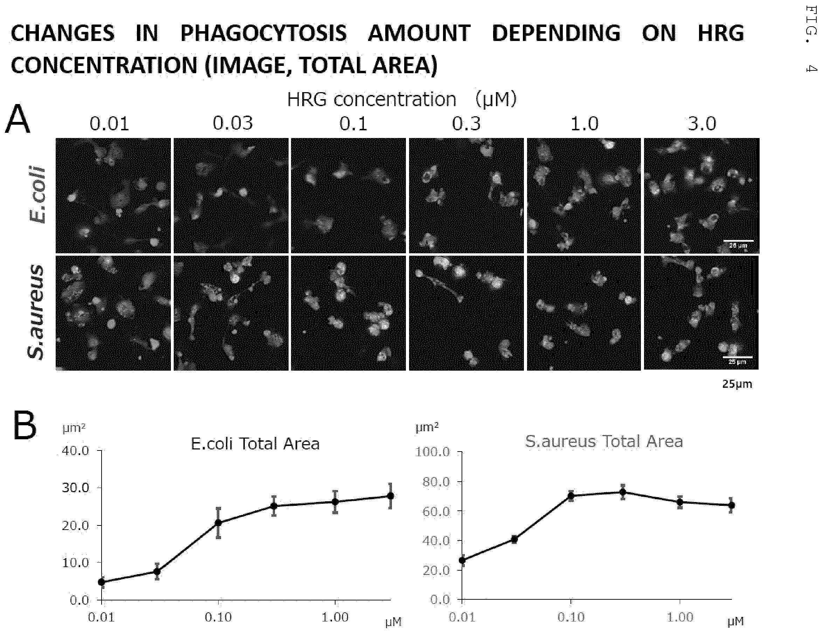

Through use of InCell Analyzer (GE Healthcare), the amounts of red-colored pHrodo by neutrophils' uptake were analyzed by image analysis and quantification to observe the phagocytosis ability of neutrophils. The analysis results of phagocytosis ability of neutrophils against *Escherichia coli* and *Staphylococcus aureus* are shown in FIG. 4A and FIG. 4B.

The results reveal that HRG enhanced phagocytosis ability of neutrophils against *Escherichia coli* and *Staphylococcus aureus.* HRG is generally contained at about 1 µM in blood, and the phagocytosis ability with HRG in this Example reached a plateau at 0.3 µM, suggesting that bacterium phagocytosis ability of neutrophils in normal individuals' blood is constantly maintained and that such phagocytosis ability is lowered in conditions with lowered HRG, such as sepsis.

### (Experimental Example 2) Observation Test 2 on Phagocytosis Ability of Neutrophils with HRG Stimulus

To the neutrophil liquids at the cell concentration (2×10⁶ cells/mL), each solution of hHRG, rHRG, or HRG-Fc was added to observe the phagocytosis ability of neutrophils against *Escherichia coli* or *Staphylococcus aureus* by the same procedure as Experimental Example 1 (HRG final concentrations of from 0.1 µM to 3.0 µM). As an observation experiment, phagocytosis ability of neutrophils against *Escherichia coli* or *Staphylococcus aureus* was observed, when each of different anti-HRG antibodies or control antibodies was added. The amounts of red-colored pHrodo by neutrophils ' uptake can be imaged and quantified to observe the phagocytosis ability of neutrophils.

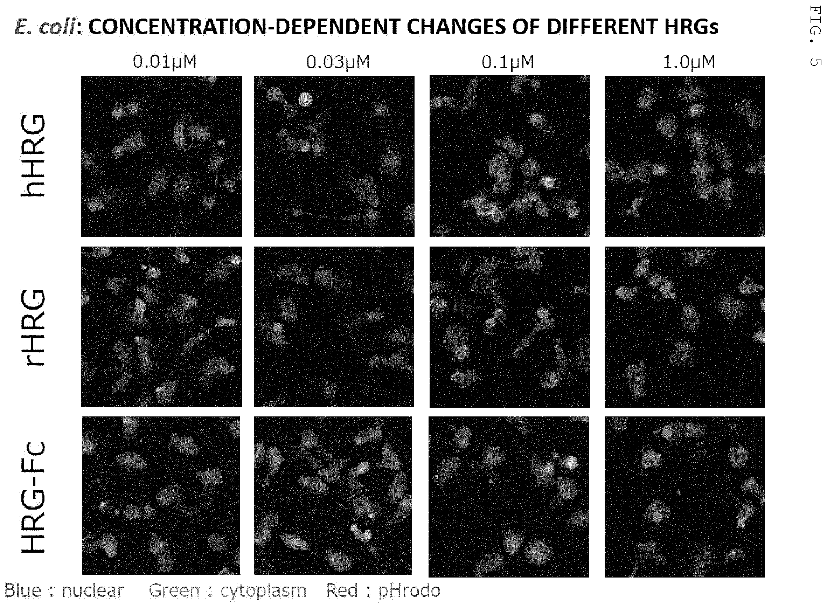

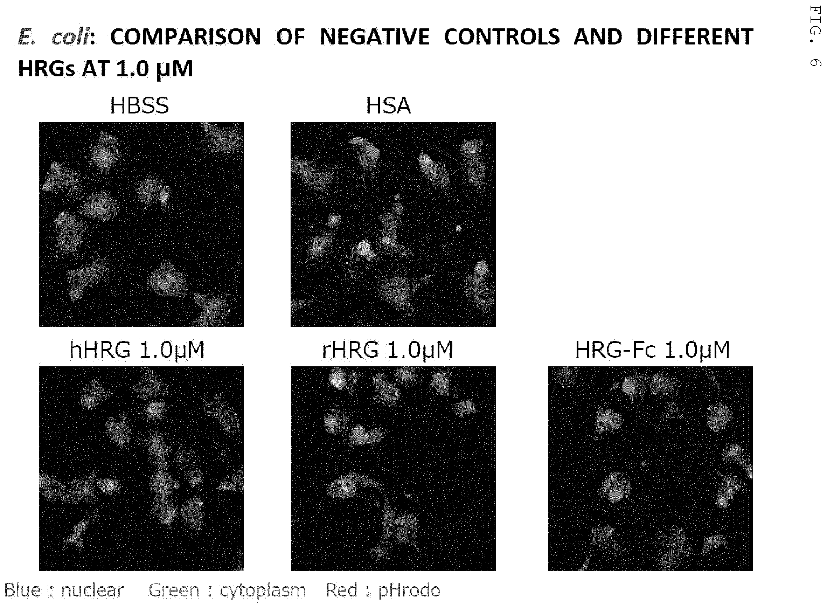

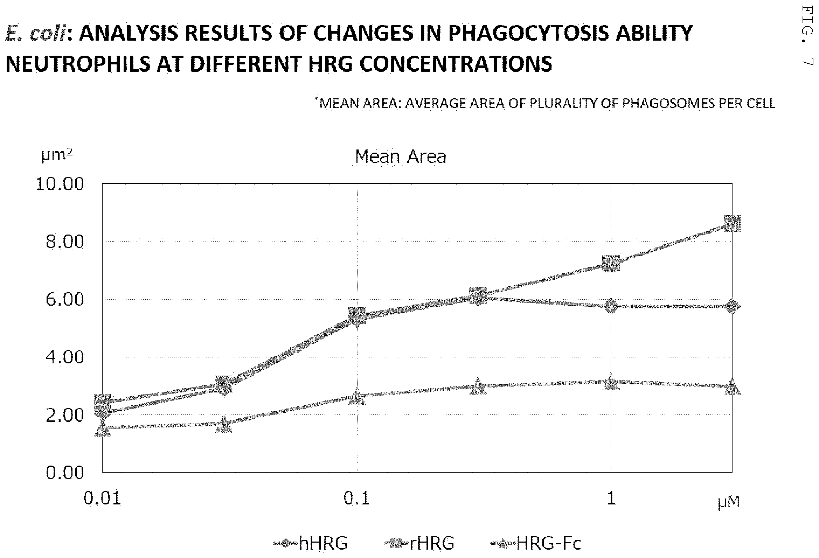

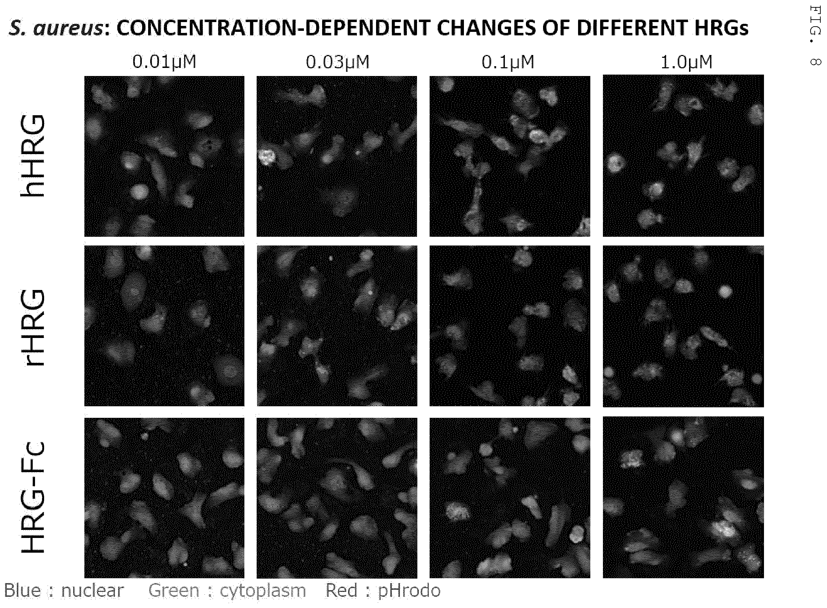

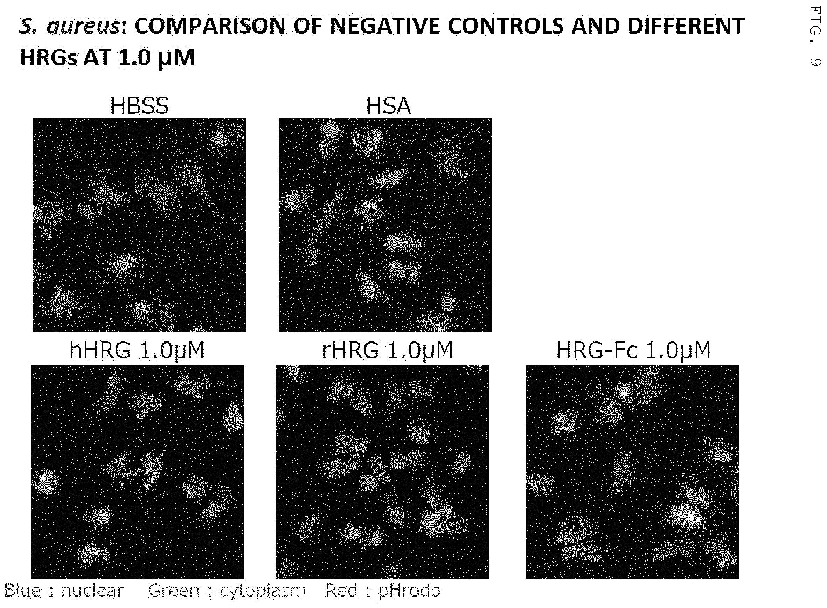

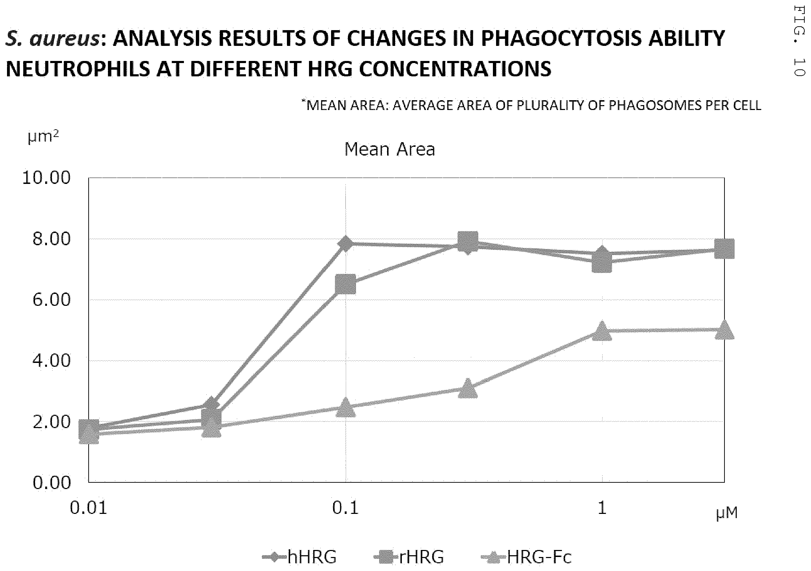

FIG. 5 and FIG. 6 show results of image analysis of phagocytosis ability of neutrophils against *Escherichia coli,* and FIG. 7 shows the change in phagocytosis ability at different concentrations and the results of analysis. Similarly, FIG. 8 and FIG. 9 show results of image analysis of phagocytosis ability of neutrophils against *Staphylococcus aureus,* and FIG. 10 shows the change in phagocytosis ability at different HRG concentrations and the results of analysis.

The results reveal that rHRG enhances phagocytosis of neutrophils against *Escherichia coli* or *Staphylococcus aureus* to the same extent as hHRG, whereas HRG-Fc is weak in phagocytosis-enhancing ability.

### (Comparative Example 1) Observation Test on Phagocytosis Ability of Neutrophils with Different Stimuli

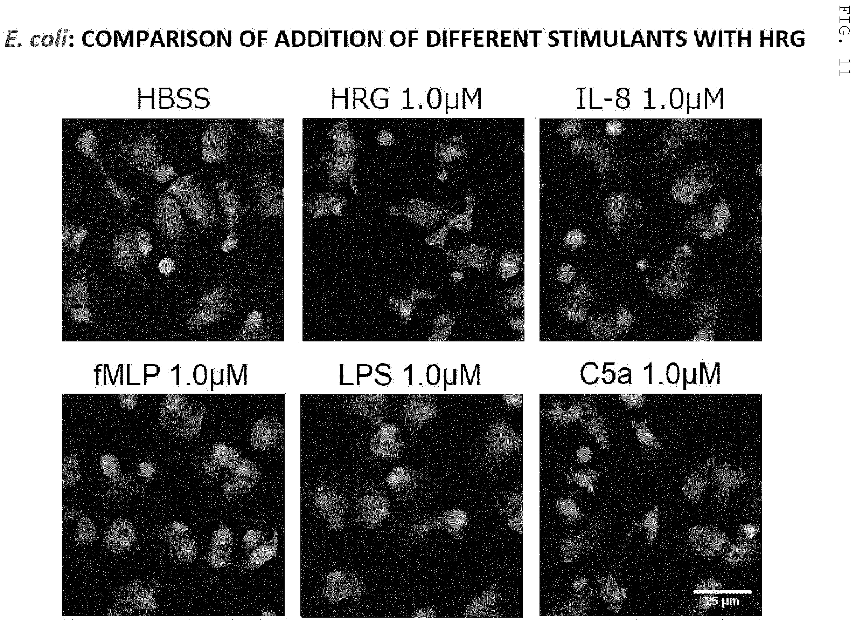

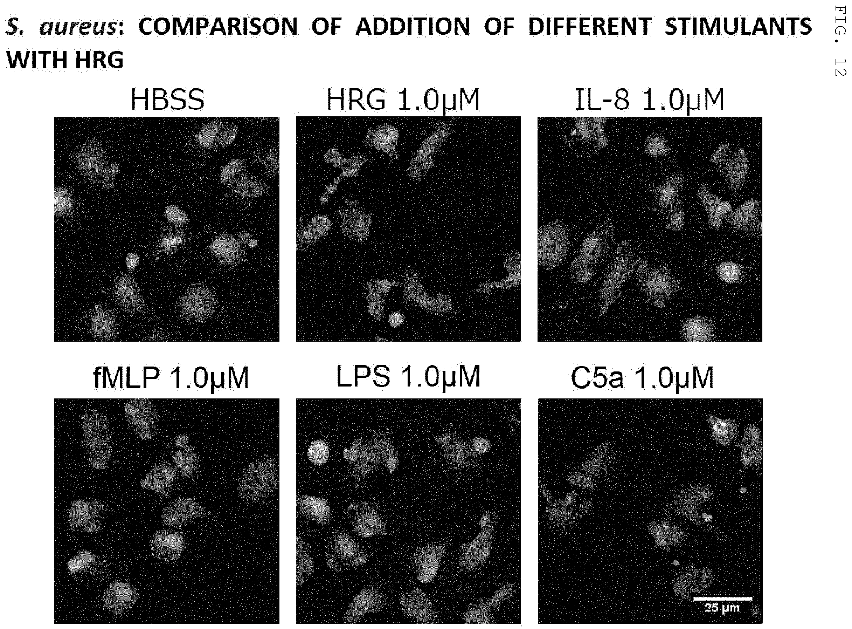

Phagocytosis ability of neutrophils against *Escherichia coli* or *Staphylococcus aureus* was observed, when 1.0 µM of hHRG was added to neutrophil solutions at the cell concentration (2×10⁶ cells/mL) by the same procedure as Example 1 or when each of 1.0 µM of IL-8, fMLP, and C5a or 10 µM of LPS was added as Comparative Example. The results of image analysis are shown in FIG. 11 and FIG. 12.

### (Experimental Example 3) Observation Test 3 on Phagocytosis Ability of Neutrophils with HRG Stimulus

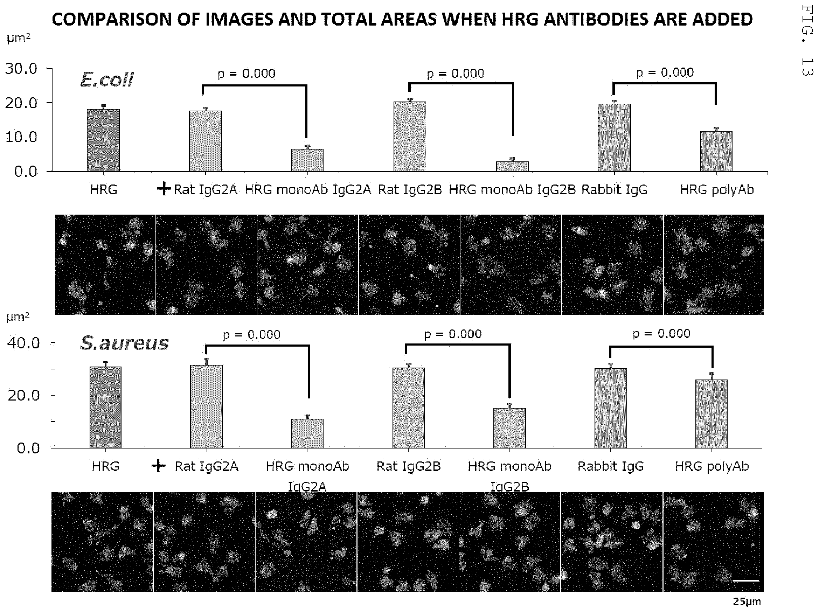

Phagocytosis ability of neutrophils against *Escherichia coli* or *Staphylococcus aureus* was observed, when 1.0 µM of hHRG was added to neutrophil solutions at the cell concentration (2×10⁶ cells/mL) by the same procedure as Example 1 or when each of 10 µM of different anti-HRG antibodies or control antibodies was added as an observation experiment. The results of analysis are shown in FIG. 13.

HRG enhanced the phagocytosis ability of neutrophils against *Escherichia coli* or *Staphylococcus aureus,* whereas the addition of each anti-HRG antibody suppressed phagocytosis ability. In contrast, the addition of the control antibodies maintained the phagocytosis ability of neutrophils against *Escherichia coli* or *Staphylococcus aureus.* This reveals that HRG has an action of enhancing the phagocytosis ability of neutrophils.

### (Experimental Example 4) Opsonin Effect 1 with HRG Stimulus

In this Experimental Example, the effect of HRG on opsonization (Op) was observed using serum containing HRG or no HRG. Healthy human serum was used for opsonization (Op). In this Experimental Example, the "serum containing HRG" refers to untreated, healthy human serum. In this Experimental Example, the "serum containing no HRG" refers to human serum in which a Ni-NTA agarose gel is added to human serum to adsorb HRG in the serum, and then the Ni-NTA agarose gel is removed by centrifugation. A Western blotting revealed that no HRG was detected in the serum containing no HRG.

Zymosan (pHrodo™ Red Zymosan, manufactured by Thermo Fisher Scientific Inc.) was blended with serum containing HRG or no HRG at 4°C, and then the mixture was stirred well by ultrasonic homogenization. After the stirring, centrifugation was performed at 4°C and 15,000 rpm for 15 min. The supernatant was discarded and the sediment was suspended in HBSS. This washing procedure was repeated twice, and then Zymosan was suspended in HBSS. The concentration of Zymosan at this time of point was 1.0 mg/mL.

To the neutrophil liquids, hHRG solutions were added, and the mixtures were dispensed to a 96-well plate, allowing incubation under CO₂ at 37°C for 30 min. Then, the Zymosan solution was added to the 96-well plate, which was then incubated under CO₂ at 37°C for 120 min. The final concentration of the cell liquids at this time was 2×10⁴ cells/well (2×10⁵ cells/mL), the final concentration of HRG was 1.0 µM, and the final concentration of Zymosan was 50 µg/well (500 µg/mL). Through use of InCell Analyzer (GE Healthcare), the amounts of red-colored pHrodo by neutrophils' uptake were analyzed by image analysis and quantification to observe the phagocytosis ability of neutrophils.

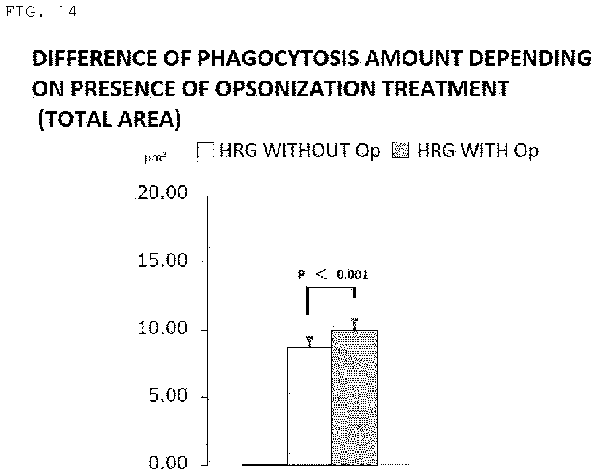

The result is shown in FIG. 14. When Zymosan was treated with the serum containing HRG or no HRG, the amount of phagocytosis by neutrophils when Zymosan was treated with the serum containing HRG was larger than that when Zymosan was treated with the serum containing no HRG, recognizing an opsonin (Op)-enhancing effect of HRG.

### (Experimental Example 5) Opsonin Effect 2 with HRG Stimulus

*Escherichia coli* (pHrodo™ Escherichia *coli*) or *Staphylococcus aureus* (pHrdo™ *Staphylococcus aureus)* was pre-incubated with human serum albumin (HSA, 1.0 µM), hHRG (1.0 µM), or 5-fold-diluted human serum (1/5 Serum) at 37°C for 15 min, and then *Escherichia coli* or *Staphylococcus aureus* was washed twice with HBSS. Then, an opsonin (Op)-enhancing effect of HRG against *Escherichia coli* or *Staphylococcus aureus* was observed by the same procedure as Example 1.

A hHRG solution was added to a neutrophil solution. The mixture was dispensed to a 96-well plate and incubated under CO₂ at 37°C for 30 min. Then, the *Escherichia coli* or *Staphylococcus aureus* pretreated with the above-mentioned HSA or HRG solution, or 5-fold-diluted human serum was added, and the resulting mixture was dispensed to the 96-well plate and incubated under CO₂ at 37°C for 120 min. The final concentration of the cell liquid at this time was 2×10⁴ cells/well (2×10⁵ cells/mL), the final concentration of HSA or HRG was 1.0 µM, and the final concentration of Zymosan was 25 µg/well (250 µg/mL). Through use of InCell Analyzer (GE Healthcare), the amounts of red-colored pHrodo by neutrophils' uptake were analyzed by image analysis and quantification to observe the phagocytosis ability of neutrophils.

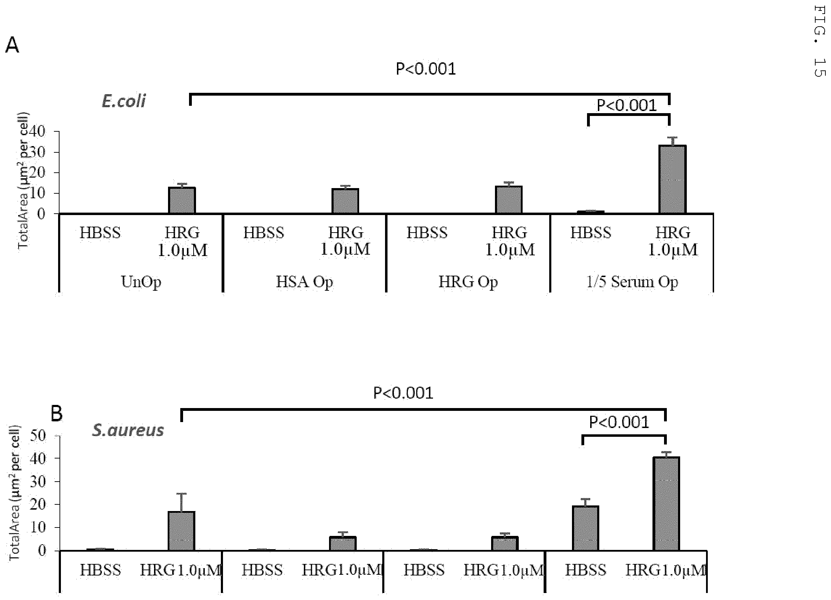

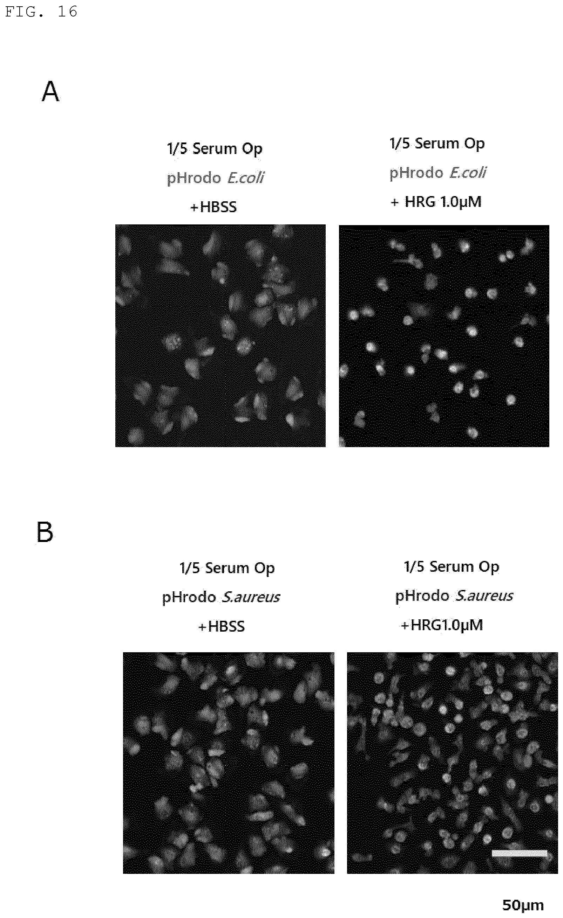

FIG. 15A and FIG. 15B show results of analysis of the phagocytosis ability of neutrophils with HRG against each of the pretreated *Escherichia coli* and *Staphylococcus aureus.* FIG. 16A and FIG. 16B show results of analysis of phagocytosis ability of neutrophils against *Escherichia coli* and *Staphylococcus aureus* pretreated with the 5-fold-diluted human serum.

In the results of this Experimental Example, the HRG pretreatment did not exhibit an opsonin (Op)-enhancing effect against *Escherichia coli* or *Staphylococcus aureus.* In contrast, the treatment with the 5-fold-diluted human serum exhibited a strong opsonin (Op) enhancement effect against *Escherichia coli* and *Staphylococcus aureus.* The HRG-pretreated bacterial cells did not enhance the phagocytosis ability of neutrophils, implying that HRG affects neutrophils rather than bacterial cells to enhance their phagocytosis ability. The phagocytosis ability of neutrophils of the bacterial cells opsonized (Op) with the human sera was highly enhanced by the addition of HRG as compared to un-opsonized (UnOp) bacterial cells, suggesting that phagocytosis actions of neutrophils against the opsonized (Op) bacterial cells in living bodies are further enhanced in the presence of HRG.

### Industrial Applicability

As described above in detail, the "agent for enhancing phagocytosis ability of neutrophils" of the present invention can enhance phagocytosis ability of neutrophils, and thus would be used as a therapeutic agent or therapeutic adjunct for various bacterial infectious diseases, viral infectious diseases, fungal infectious diseases, parasitic infectious diseases, and mixed infectious diseases thereof. The "agent for enhancing phagocytosis ability of neutrophils" of the present invention would be used with any other pharmaceutical formulation, for example, a therapeutic agent already put on the market or a therapeutic agent to be developed in the future, allowing the reduction in dose/administration frequency of the other pharmaceutical formulation. When the other pharmaceutical formulation has a side effect, the combined use of the other pharmaceutical formulation and the "agent for enhancing phagocytosis ability of neutrophils" of the present invention can reduce the dose/administration frequency of the other pharmaceutical formulation, which is useful.

## Claims

1. An agent for enhancing phagocytosis ability of neutrophils, comprising a histidine-rich glycoprotein as an active ingredient.

2. The agent for enhancing phagocytosis ability of neutrophils according to claim 1, wherein the histidine-rich glycoprotein, which is the active ingredient, is present in the form of an Fc-fusion protein comprising the histidine-rich glycoprotein fused with an Fc region of an antibody.

3. The agent for enhancing phagocytosis ability of neutrophils according to claim 2, wherein the Fc region of an antibody is an Fc region derived from IgG₂.

4. The agent for enhancing phagocytosis ability of neutrophils according to any one of claims 1 to 3, wherein the agent serves as a therapeutic agent for an infectious disease or a therapeutic adjunct for an infectious disease.

5. The agent for enhancing phagocytosis ability of neutrophils according to claim 4, wherein the infectious disease is an infectious disease caused by a pathogen derived from one or a plurality of species selected from bacteria, viruses, fungi, and parasites.

6. The agent for enhancing phagocytosis ability of neutrophils according to claim 4 or 5, wherein the infectious disease is any one of infectious diseases selected from a respiratory infectious disease, a urinary tract infectious disease, a biliary tract infectious disease, a gastrointestinal infectious disease, and a central nervous system infectious disease.


SEQUENCE LISTING

<110>  National University Corporation Okayama University

<120>  Phagocytosis enhancer of Neutrophils

<130>  GP19-1009PCT

<150>  JP P2018-123626
<151>  2018-06-28

<160>  4

<170>  PatentIn version 3.5

<210>  1
<211>  507
<212>  PRT
<213>  Homo sapiens

<400>  1

Val Ser Pro Thr Asp Cys Ser Ala Val Glu Pro Glu Ala Glu Lys Ala
1               5                   10                  15

Leu Asp Leu Ile Asn Lys Arg Arg Arg Asp Gly Tyr Leu Phe Gln Leu
            20                  25                  30

Leu Arg Ile Ala Asp Ala His Leu Asp Arg Val Glu Asn Thr Thr Val
        35                  40                  45

Tyr Tyr Leu Val Leu Asp Val Gln Glu Ser Asp Cys Ser Val Leu Ser
    50                  55                  60

Arg Lys Tyr Trp Asn Asp Cys Glu Pro Pro Asp Ser Arg Arg Pro Ser
65                  70                  75                  80

Glu Ile Val Ile Gly Gln Cys Lys Val Ile Ala Thr Arg His Ser His
                85                  90                  95

Glu Ser Gln Asp Leu Arg Val Ile Asp Phe Asn Cys Thr Thr Ser Ser
            100                 105                 110

Val Ser Ser Ala Leu Ala Asn Thr Lys Asp Ser Pro Val Leu Ile Asp
        115                 120                 125

Phe Phe Glu Asp Thr Glu Arg Tyr Arg Lys Gln Ala Asn Lys Ala Leu
    130                 135                 140

Glu Lys Tyr Lys Glu Glu Asn Asp Asp Phe Ala Ser Phe Arg Val Asp
145                 150                 155                 160

Arg Ile Glu Arg Val Ala Arg Val Arg Gly Gly Glu Gly Thr Gly Tyr

                165                 170                 175

Phe Val Asp Phe Ser Val Arg Asn Cys Pro Arg His His Phe Pro Arg
            180                 185                 190

His Pro Asn Val Phe Gly Phe Cys Arg Ala Asp Leu Phe Tyr Asp Val
        195                 200                 205

Glu Ala Leu Asp Leu Glu Ser Pro Lys Asn Leu Val Ile Asn Cys Glu
    210                 215                 220

Val Phe Asp Pro Gln Glu His Glu Asn Ile Asn Gly Val Pro Pro His
225                 230                 235                 240

Leu Gly His Pro Phe His Trp Gly Gly His Glu Arg Ser Ser Thr Thr
                245                 250                 255

Lys Pro Pro Phe Lys Pro His Gly Ser Arg Asp His His His Pro His
            260                 265                 270

Lys Pro His Glu His Gly Pro Pro Pro Pro Pro Asp Glu Arg Asp His
        275                 280                 285

Ser His Gly Pro Pro Leu Pro Gln Gly Pro Pro Pro Leu Leu Pro Met
    290                 295                 300

Ser Cys Ser Ser Cys Gln His Ala Thr Phe Gly Thr Asn Gly Ala Gln
305                 310                 315                 320

Arg His Ser His Asn Asn Asn Ser Ser Asp Leu His Pro His Lys His
                325                 330                 335

His Ser His Glu Gln His Pro His Gly His His Pro His Ala His His
            340                 345                 350

Pro His Glu His Asp Thr His Arg Gln His Pro His Gly His His Pro
        355                 360                 365

His Gly His His Pro His Gly His His Pro His Gly His His Pro His
    370                 375                 380

Gly His His Pro His Cys His Asp Phe Gln Asp Tyr Gly Pro Cys Asp
385                 390                 395                 400

Pro Pro Pro His Asn Gln Gly His Cys Cys His Gly His Gly Pro Pro
                405                 410                 415

Pro Gly His Leu Arg Arg Arg Gly Pro Gly Lys Gly Pro Arg Pro Phe
            420                 425                 430

His Cys Arg Gln Ile Gly Ser Val Tyr Arg Leu Pro Pro Leu Arg Lys
        435                 440                 445

Gly Glu Val Leu Pro Leu Pro Glu Ala Asn Phe Pro Ser Phe Pro Leu
    450                 455                 460

Pro His His Lys His Pro Leu Lys Pro Asp Asn Gln Pro Phe Pro Gln
465                 470                 475                 480

Ser Val Ser Glu Ser Cys Pro Gly Lys Phe Lys Ser Gly Phe Pro Gln
                485                 490                 495

Val Ser Met Phe Phe Thr His Thr Phe Pro Lys
            500                 505

<210> 2
<211> 227
<212> PRT
<213> Homo sapiens

<400> 2

Glu Arg Lys Cys Cys Val Glu Cys Pro Pro Cys Pro Ala Pro Pro Val
1               5                   10                  15

Ala Gly Pro Ser Val Phe Leu Phe Pro Pro Lys Pro Lys Asp Thr Leu
            20                  25                  30

Met Ile Ser Arg Thr Pro Glu Val Thr Cys Val Val Val Asp Val Ser
        35                  40                  45

His Glu Asp Pro Glu Val Gln Phe Asn Trp Tyr Val Asp Gly Val Glu
    50                  55                  60

Val His Asn Ala Lys Thr Lys Pro Arg Glu Glu Gln Phe Asn Ser Thr
65                  70                  75                  80

Phe Arg Val Val Ser Val Leu Thr Val Val His Gln Asp Trp Leu Asn
                85                  90                  95

Gly Lys Glu Tyr Lys Cys Lys Val Ser Asn Lys Gly Leu Pro Ala Pro
            100                 105                 110

Ile Glu Lys Thr Ile Ser Lys Thr Lys Gly Gln Pro Arg Glu Pro Gln
        115                 120                 125

Val Tyr Thr Leu Pro Pro Ser Arg Glu Glu Met Thr Lys Asn Gln Val
    130                 135                 140

Ser Leu Thr Cys Leu Val Lys Gly Phe Tyr Pro Ser Asp Ile Ser Val
145                 150                 155                 160

Glu Trp Glu Ser Asn Gly Gln Pro Glu Asn Asn Tyr Lys Thr Thr Pro
                165                 170                 175

Pro Met Leu Asp Ser Asp Gly Ser Phe Phe Leu Tyr Ser Lys Leu Thr
            180                 185                 190

Val Asp Lys Ser Arg Trp Gln Gln Gly Asn Val Phe Ser Cys Ser Val
        195                 200                 205

Met His Glu Ala Leu His Asn His Tyr Thr Gln Lys Ser Leu Ser Leu
    210                 215                 220

Ser Pro Gly
225

<210>  3
<211>  1596
<212>  DNA
<213>  Artificial

<220>
<223>  EcoR1-HRG-Xho1

<400>  3
gaattccgga tggggaaggc actcattgca gcactgcttt tgatcacatt gcagtattcg      60

tgtgccgtga gtcccactga ctgcagtgct gttgagccgg aggctgagaa agctctagac     120

ctgatcaata aaaggcgacg ggatggctac cttttccaat tgctgcggat tgctgatgcc     180

cacttggaca gagtggaaaa tacaactgta tattacttag tcttagatgt gcaagaatcg     240

gactgttcgg tcctatccag gaaatactgg aatgactgtg agccacctga ttccagacgt     300

ccatctgaaa tagtgatcgg acaatgtaag gtaatagcta caagacattc ccatgaatct     360

caggacctca gagtgattga ctttaactgc accacaagtt ctgtctcttc agcactggcc     420

aataccaaag atagtccggt cctcatagat ttctttgagg atactgagcg ctacagaaaa     480

caagccaaca aagcccttga gaagtacaaa gaggagaatg atgactttgc ctctttcaga     540

gtggaccgaa tcgagagagt tgcaagagtg agaggagggg aaggaactgg ttacttcgtg     600

gacttctctg tgcggaactg ccccagacac catttcccca gacaccccaa tgtctttgga     660

ttctgcagag cagatttgtt ctatgatgta gaagccttgg acttggaaag cccgaaaaac     720

cttgtcataa actgtgaagt cttcgaccct caggaacatg agaacatcaa tggtgtaccg     780

cctcatttgg gacatccctt ccactggggt gggcatgagc gttcttctac caccaagcct 840

ccattcaagc cccatggatc tagagatcat catcatcccc acaagccaca cgaacatgga 900

cccccacctc ctccagatga aagagatcac tcacatggac ccccacttcc acaaggccct 960

cctccactat tgcccatgtc ctgctcaagt tgtcaacatg ccacttttgg cacaaatggg 1020

gcccaaagac attctcataa taataattcc agtgacctcc atccccataa gcatcattcc 1080

catgaacagc atccccacgg acaccatccc catgcacacc atcctcatga acatgatacc 1140

catagacagc atccccatgg acaccacccc catggacacc atcctcatgg acaccacccc 1200

catggacacc atccccatgg acaccatccc cactgccatg atttccaaga ctatggacct 1260

tgtgacccac caccccataa ccaaggtcac tgttgccatg gccacggccc accacctggg 1320

cacttaagaa ggcgaggccc aggtaaagga ccccgtccct tccattgcag acaaattgga 1380

tctgtgtacc gactccctcc tctaagaaaa ggtgaggtgc tgccacttcc tgaggccaat 1440

tttcccagct tcccattgcc gcaccacaaa catcctctaa agccagacaa tcagcccttt 1500

cctcaatcag tctctgaatc atgtccaggg aagttcaaga gtgggtttcc acaagtttcc 1560

atgtttttta cacatacatt tccaaaaccg ctcgag 1596

<210> 4
<211> 722
<212> DNA
<213> Artificial

<220>
<223> XhoI-GPG-hIgG2 Fc-Xba1

<400> 4
ctcgagcggg gacctggaga gcgcaaatgt tgtgtcgagt gcccaccgtg cccagcacca 60

cctgtggcag gaccgtcagt cttcctcttc cccccaaaac ccaaggacac cctcatgatc 120

tcccggaccc ctgaggtcac gtgcgtggtg gtggacgtga gccacgaaga ccccgaggtc 180

cagttcaact ggtacgtgga cggcgtggag gtgcataatg ccaagacaaa gccacgggag 240

gagcagttca acagcacgtt ccgtgtggtc agcgtcctca ccgtcgtgca ccaggactgg 300

ctgaacggca aggagtacaa gtgcaaggtc tccaacaaag gcctcccagc ccccatcgag 360

aaaaccatct ccaaaaccaa agggcagccc cgagaaccac aggtgtacac cctgccccca 420

tcccgggagg agatgaccaa gaaccaggtc agcctgacct gcctggtcaa aggcttctac 480

cccagcgaca tctccgtgga gtgggagagc aatgggcagc cggagaacaa ctacaagacc 540

acacctccca tgctggactc cgacggctcc ttcttcctct acagcaagct caccgtggac 600

aagagcaggt ggcagcaggg gaacgtcttc tcatgctccg tgatgcatga ggctctgcac 660

aaccactaca cacagaagag cctctccctg tctccgggta aatgactaac tagcgctcta 720

ga 722